# EUROPÄISCHE PATENTSCHRIFT

(11) **EP 1 128 859 B1**
(45) Veröffentlichungstag und Bekanntmachung des Hinweises auf die Patenterteilung: **21.09.2005**
(21) Anmeldenummer: 99970614.6
(22) Anmeldetag: 18.10.1999
(51) Int. Cl.: A61M 5/30

(54) **DRUCKSTRAHL-INJEKTOR ZUM SCHMERZLOSEN INJIZIEREN VON MEDIKAMENTEN**
PRESSURE JET INJECTOR FOR PAINLESSLY INJECTING MEDICAMENTS
DISPOSITIF D'INJECTION PAR JET SOUS PRESSION POUR L'INJECTION SANS DOULEUR DE MEDICAMENTS

(30) Priorität: 16.10.1998 DE 19849301; 16.10.1998 DE 29823308 U
(43) Veröffentlichungstag der Anmeldung: 05.09.2001
(73) Patentinhaber: Kolbe, Eckard, 12277 Berlin (DE)
(72) Erfinder: KOLBE, Eckert, D-15831 Grossziethen (DE); LAWRENZ, Horst, D-10789 Berlin (DE); KERSTEN, Jens, D-15344 Strausberg (DE)
(86) Internationale Anmeldenummer: PCT/EP1999/007863
(87) Internationale Veröffentlichungsnummer: WO 2000/023132

(56) Entgegenhaltungen:
- EP-A- 0 800 841
- GB-A- 2 307 860
- US-A- 5 505 697

## Beschreibung

Die Erfindung betrifft einen Druckstrahl-Injektor zum schmerzlosen Injizieren von Medikamenten gemäß dem Oberbegriff des Anspruchs 1.

In der DE 195 19 278 A1 werden ein Druckstrahlinjektor und ein Verfahren zur Behandlung von Diabetikern unter Anwendung der nadellosen Druckstrahlinjektion beschrieben, bei denen eine Saugglocke zur hautschonenden Injektion zum Einsatz kommt. Es wird dabei unter Verwendung von Rechentechnik vorgeschlagen, eine optische Überprüfung der Haut auf Punktionseignung vorzunehmen und die natürliche Hautfelderung als Maßstab zu benutzen. Die Reflexionsbeschaffenheit der Injektionsstelle wird mit einer benachbarten Hautstelle verglichen. Aus dem Vergleich wird deren Eignung abgeleitet.

Dieses Verfahren berücksichtigt nicht die individuelle Hautbeschaffenheit in ihrem Aufbau, sondern nur deren optische Oberflächeneigenschaft. Es werden keine Zusammenhänge zwischen den Parametern der vorzunehmenden Injektion und der Hautbeschaffenheit hergestellt, so daß keine optimale Injektion gewährleistet werden kann und auch Beschädigungen der Haut durch zum Beispiel einem zu hohen Injektionsdruck nicht auszuschließen sind. Durch Hautbeschädigungen besteht die Gefahr von Infektionen wie Aids über austretende Gewebeflüssigkeit oder Bluttröpfchen.

Der Erfindung liegt die Aufgabe zugrunde, einen Druckstrahl-Injektor der eingangs genannten Art zu entwickeln, mit denen Medikamente in flüssiger Form in optimaler Dosierung zuverlässig, auf die Bedingungen des Patienten abgestimmt und leicht zu bedienen injiziert werden können, und mit denen gewährleistet ist, daß Gewebeschäden und damit Infektionen weitestgehend ausgeschlossen sind.

Erfindungsgemäß wird diese Aufgabe durch die Merkmale des Anspruchs 1 gelöst. Durch die Regelung des Injektionsdruckes und der Injektionssignalform in Abhängigkeit von dem an der Injektionsstelle gemessenen elektrischen Hautwiderstand und der ermittelten Hautelastizität, derart, daß die zu injizierende Flüssigkeit durch Variierung des Injektionsdrucks in eine bestimmte Schicht der Haut injiziert wird, wobei die Menge des zu injizierenden Medikamentes aus Patienteneingaben, Voreinstellungen des behandelnden Arztes sowie aus aktuellen Messungen errechnet wird, können optimale Bedingungen für die Injektion geschaffen werden, die Schmerzfreiheit und die Vermeidung von Schädigungen und Infektionen durch die Injektion gewährleisten. Die Einstellungen des Patienten in der Medikation erfolgen optimal. Das Injektionsgebiet wird schonend ausgewählt, Mißbildungen der Haut werden als Injektionsgebiet ausgesondert. Das für eine Injektion geeignete Gebiet wird gegenüber den herkömmlichen Verfahren wesentlich erweitert. Injektionsdaten wie Injektionsmengen, Injektionsinter-valle und Aktivitäten des Patienten können aufeinander abgestimmt werden. Der behandelnde Arzt kann über einen längeren Zeitraum die Wirkungen der Medikation überschauen und Rückschlüsse ziehen und frühzeitig auf Veränderungen reagieren.

Das Injektionsgerät sieht Einrichtungen zur Messung des elektrischen Hautwiderstandes und zur Ermittlung der Hautelastizität, die mit einer Rechnereinheit wie Mikroprozessor zur Auswertung der Meßergebnisse und zur Bestimmung und Variierung des Injektionsdruckes verbunden sind, eine Dosiereinrichtung mit Rückkopplung zur Rechnereinheit zur Verwaltung der zur Anwendung vorgesehenen Medikamente, eine Ansaugeinrichtung mit Rückkopplung zur Rechnereinheit zur Vorbereitung des Injektionsbereiches und eine Abschußeinrichtung mit Rückkopplung zur Rechnereinheit zur zeit- und kraftgenauen Injektion vor, wobei die Rechnereinheit mit Einrichtungen zur Dateneingabe und Datenausgabe verbunden ist.

Vorteilhafte Weiterbildungen der Erfindung ergeben sich aus den Unteransprüchen.

Die Erfindung soll nachstehend an einem Ausführungsbeispiel eines Druckstrahl-Injektors näher erläutert werden. In der zugehörigen Zeichnung zeigen:
- Fig. 1: eine schematische Darstellung der Baugruppen des Injektionsgerätes,
- Fig. 2: die grafische Darstellung des Signalverlaufs des Ausgangsdruckes und
- Fig. 3: die schematische Darstellung des Saugglockenkopfes mit angesaugter Haut.

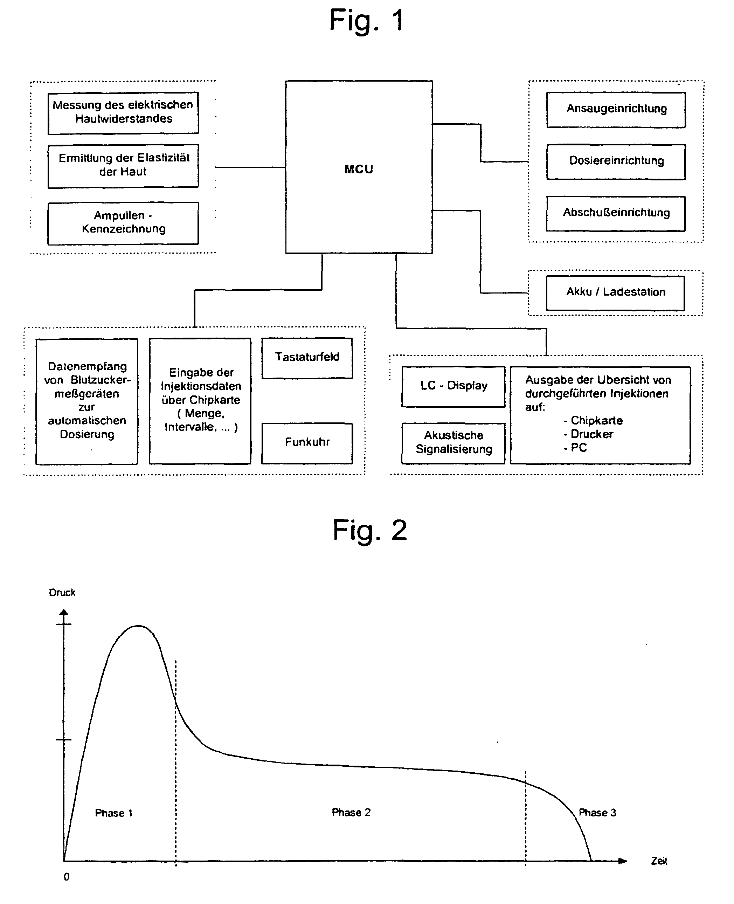

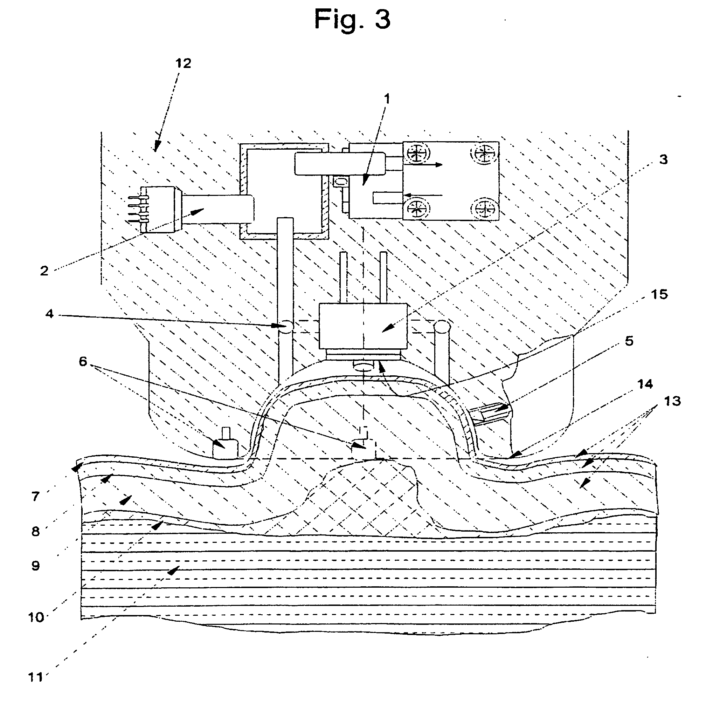

Entsprechend der Darstellung in der Fig. 1 besteht das Injektionsgerät nach der Erfindung im wesentlichen aus den Baugruppen Ansaugeinrichtung, Dosiereinrichtung, Abschußeinrichtung, Stromversorgung, Hautwiderstandsmessung, Hautelastizitätsmessung, Ampullenkennzeichnung, Dateneingabe, Datenausgabe, Rechnereinheit (Mikroprozessoreinheit) und einem zum Teil in der Fig. 3 dargestellten Leitungssystem mit Ventilsteuerung.

Um eine Injektion weitestgehend schmerzlos erfolgen zu lassen, ist gemäß der Darstellung in der Fig. 3 mittels einer Saugglocke 12 und einer Saugpumpe 1 ein für die Haut 13 ungefährlicher Unterdruck von max. 200 mbar für max. 5 s zu erzeugen. Damit wird das ausgewählte Hautareal angesaugt und gestrafft, was zur Folge hat, daß das Zielgebiet der beabsichtigten Injektion von größeren Gefäßen und Nerven abgehoben wird und günstig vor einer Abschußdüse 5 liegt.

Die Ansaugeinrichtung nach Fig. 1 besteht im wesentlichen aus der Saugglocke 12 (Fig. 3), deren Ausformung halbkugel- bis linsenförmig ist. Die rundum verlaufende Kante 14 ist zum Schutz der anzusaugenden Haut 13 stark abgerundet. Der Durchmesser der Saugglocke 12 kann zwischen 25 mm und 30 mm und die Tiefe zwischen 11 mm bis 17 mm gewählt sein. Zwecks Überwachung der ca. 5 s andauernden Ansaugung sind innerhalb und außerhalb der Ansaugeinrichtung Überwachungssensoren 2,3,6 angebracht. Zu den Überwachungs-sensoren gehört ein Sensor 6, der den elektrischen Hautwiderstand mißt, sowie ein Luftdrucksensor 2, der in Verbindung mit einem optischen Entfernungsmeßsensor 3 die Hautelastizität bestimmt. Der elektrische Widerstandsmeßsensor 6 befindet sich am unteren Rand 14, der optische Entfernungsmeßsensor 3 mittig in der Saugglocke 12 und der Luftdrucksensor 2 mit der Ansaugpumpe 1 im Inneren des Saugglockenkörpers 12. Die Abschußdüse 5 für das Injektionsmittel befindet sich ebenfalls in der Saugglocke 12. Sie ist so angebracht, daß sich die Haut 13 ab einer bestimmten Ansaughöhe direkt an die Düse 5 anlegt.

Bis kurz vor dem Abschuß kommuniziert die Ansaugeinrichtung mittels der Überwachungssensoren 2,3,6 zwecks hautschonender Nachregelung mit der Rechnereinheit MCU. Nach erfolgter Injektion wird durch Öffnen eines Belüftungsventiles im Saugglockenraum 12 der Innendruck dem Außendruck über ein Luftansaugringleitungssystem 4 angeglichen.

Mit der Dosiereinrichtung nach Fig. 1 werden durch die Rückkopplung zur Rechnereinheit MCU sämtliche zur Anwendung kommenden Präparate aus dem Vorrat bereitgestellt und diese mit einer definierten Auflösung und einer definierten hohen Genauigkeit an die Abschußeinrichtung übergeben.

Voraussetzung dafür ist das Vorhandensein eines geeignetes Vorratsbehältersystems (Ampullen) im Verbund mit Ventilen und Leitungen.
Um eine Manipulation und Kontaminierung des Inhalts der Vorratsbehälter auszuschließen, sind Vorratsbehälter als Einweg- bzw. als Pfandbehälter vorgesehen, die vom Anwender nicht befüllbar sind. Die Verwechslung von Vorratsbehältern wird mit mechanisch wirksamen Kodiermöglichkeiten und einem mit dem Vorratsbehälter integrierten Speicherchip verhindert. In diesem befinden sich die Identifikationsdaten, wie z.B. das Verfallsdatum, der ID-Code des Vorratsbehälters, die Füllmenge usw. Die mechanische Kodierung wird so ausgelegt, daß der Speicherchip nur ausgelesen werden kann, wenn der korrekte Vorratsbehälter in den dafür richtigen Aufnahmeschacht eingelegt ist. Als Vorlage für Form und Aufbau der Vorratsbehälter ist eine allgemein bekannte Insulinpatrone oder eine Mehrkammerpatrone, ähnlich einer Tintenstrahl-druckerpatrone, zur Aufnahme von Präparat und Reinigungsmittel denkbar.

Da das System luftdicht ist, wird die tatsächlich abgegebene Präparatsmenge unter Zuhilfenahme von Wegmeßsystemen am jeweiligen Vorratsbehälter und im Abschußsystem durch die Rechnereinheit MCU rechnerisch bestimmt. Der Austrieb der Präparate aus den Vorratsbehältern erfolgt durch Federkraft und Ventilbetätigung.

In einer Ausführungsform des Injektionsgerätes wird automatisch, nach Berechnung der über einen beim Patienten implantierten Mikrochip oder über ein externes Blutzuckermeßgerät erfaßten Blutzuckerwerte, die benötigte Präparatsmenge bereitgestellt.

Die Abschußeinrichtung nach Fig. 1 besteht aus den Unterkomponenten Abschußdüse 5 (Fig. 3), Abschußraum und Abschußantrieb (nicht dargestellt).

Die Abschußdüse 5 befindet sich in der Ansaugglocke 12 und ist somit das Verbindungsglied zwischen Ansaugeinrichtung und Abschußeinrichtung.

Die Düse 5 ist so konstruiert, daß das Injektionsgut als Vollstrahl, ähnlich wie in einer Injektionskanüle, gebündelt wird, und im richtigen Winkel die obersten Hautschichten 7,8 durchdringt und dann in die Subkutis 9 gelangt (Fig. 3).

Der Durchmesser der Düsenöffnung kann zwischen 0,07 mm und 0,2 mm gewählt sein. Die maximale Ausschußgeschwindigkeit beträgt ca. 300 m/s. Die Düse 5 wird für einen Betriebsdruck von etwa 550 bar ausgelegt. Als Düsenmaterial wird Stahl, Hartmetall oder Saphir verwendet.

Im Abschußraum nach Fig. 1 werden die von der Dosiereinrichtung bereitgestellten Präparate vermischt und bis zum endgültigen Abschuß aufgenommen.

Der Abschußraum ist zylindrisch und wird von einem beweglichen Abschußkolben abgeschlossen. Der Durchmesser kann 5 mm und sein Aufnahmevolumen 0,5 ml betragen. Überprüft wird die Aufnahmemenge mittels höchstauflösender Wegmeßsensoren und dem Innendurchmesser des Abschußzylinders. Die maximal zu erwartende Druckbelastung wird 550 bar nicht überschreiten. Das Ausgangssignal (Abschuß) wird mittels Drucksensor im Abschußzylinder aufgenommen. Dieser befindet sich im Abschußzylinderboden.

Durch den Vergleich der Ansteuersignale mit dem tatsächlichen Druckverlauf kann ein geeignetes Abschußantriebssystem ausgewählt werden.

Der Abschußantrieb bewegt den Abschußkolben zeit- und kraftgenau unter der Steuerung der Rechnereinheit MCU im Abschußzylinder. Seine Höchstkraft muß ca. 1050 N betragen und seine Höchstvorschubgeschwindigkeit 0,12 m/s. Die Realisierung des Abschußantriebes kann durch mehrere Varianten erfolgen:
- Hydraulikzylinder unter Zuhilfenahme einer Hydraulikpumpe,
- direkt wirkende Linearmotoren bzw. Synchron-Linearmotoren,
- Federkraftspeicher, der mittels Motor oder ähnlichem gespannt wird,
- Schrittmotoren zum Antrieb einer Zahnstange bzw. einer Spindel,
- kaskadierte Piezoaktoren etc.

Durch das Leitungssystem mit Ventilsteuerung wird der Transport des Präparates und des Reinigungsmittels zur Befüllung des Abschußraumes und der Reinigung des Gesamtsystems realisiert. Es verbindet die Dosiereinrichtung mit dem Abschußraum und der Abschußdüse 5 auf kürzestem Weg.

Der Reinigungsvorgang kann wie folgt konzipiert sein: Der Reinigungsvorgang erfolgt vor und nach jeder Injektion, wobei anhand von zeitlicher Erfassung nach längerer Injektionspause intensiver gereinigt wird. Für eine Reinigung wird Reinigungsmittel vom Vorratsbehälter in den Abschußraum geleitet. Zur Reinigung der Abschußdüse 5 wird das im Abschußraum befindliche Reinigungsmittel leistungsgemindert ausgestoßen.

Vor jeder Injektion sind an der vorgesehenen Injektionsstelle biophysikalische Untersuchungen wie Hautwiderstandsmessung und Ermittlung der Hautelastizität nach Fig. 3 vorgesehen.

Die biophysikalische Untersuchung des für eine Injektion vorgesehenen Hautbereiches stellt den Schwerpunkt der Datenaufnahme dar. Ziel dieser Untersuchung ist es, die Parameter der einzelnen Hautschichten und des darunter befindlichen Gewebes aufzunehmen. Die Injektionsstelle ist auf die Eindringtiefe, die Streuung, die Deformation- und den Transport von Zellen hin zu untersuchen. Weiterhin sind die üblichen Daten über den Probanden aufzunehmen.

Der elektrische Widerstand der Haut 13 bewegt sich zwischen ca. 1KΩ/cm² bei Kindern bis hin zu 15KΩ/cm² bei älteren Menschen. Er ist Abhängig vom Flüssigkeits- bzw. Elektrolytgehalt und der Dicke der oberen Hautschichten 7,8. Die Epidermis 7 kann zwischen 0,04 mm und 0,2 mm, die Dermis 8 zwischen ca. 0,6 mm und 3,0 mm, die Subcutis 9 bis zu 3 cm stark sein. Dies läßt den Schluß auf die Festigkeit der oberen Hautschichten 7,8 zu, denn je mehr Flüssigkeit sich in den Schichten 7,8 befindet, um so weicher sind diese. Um dies zu bestätigen, ist es erforderlich, diese Umgebung nach der elektronischen Messung hinsichtlich des biomechanischen Aufbaus zu untersuchen. Der Hautwiderstand hat somit einen direkten Einfluß auf den Injektionsdruck und dessen Signalform nach Fig. 2.

Durch gleichzeitige Messung der Leistungsaufnahme der Ansaugpumpe 1, des Ansaugdruckes und des Abstandes der Haut 13 zum Ansaugglockenboden 15 kann die Elastizität der Haut 13 bestimmt werden. Dadurch können die unteren Hautschichten 9 und das Zwischengewebe beurteilt werden. Diese Erkenntnisse können direkt (Regelung der Einsaugtiefe) oder indirekt (als Faktor) in die Berechnung des Injektionsdruckes einfließen.

Mit der Aufnahme der Druckänderung in der Injektionseinrichtung (Abschuß) wird angestrebt, eine optimale Anfangsgeschwindigkeit des zu verabreichenden Präparates von ca. 300 m/sec, da. sich bei dieser Geschwindigkeit flüssige Stoffe nahezu wie feste Körper verhalten. Die Dokumentation der Injektionssignalform ist, um die komplexen Vorgänge später in die Auswertung einfließen zu lassen. Die anzustrebende Signalform ist in der Fig. 1 dargestellt.

Danach ist in einer Phase 1 zur Öffnung der Epidermis 7 entsprechend der Darstellung in Fig. 3 während einer Zeit von ca. 10 ms der maximale Anfangsdruck aufzubauen, der von den gemessenen Hautparametern abhängig zu machen ist. In einer Phase 2 ist zur Verabreichung des Impfstoffes der gewünschte Injektionsdruck aufrechtzuerhalten. In einer Phase 3 ist der Druck auf Null abzusenken, um die Hautöffnung rückängig zu machen. Die Injektion erfolgt vorzugsweise in die Subcutis 9 in einem Winkel, der einen großen Wirkungsraum erfaßt. Durch die Injektion des Impfstoffes mit optimierten individuellen Parametern in die Subcutis 9 werden Gewebeverletzungen und damit Infektionsgefahren zuverlässig vermieden und Schmerzfreiheit gewährleistet.

Weiterhin ist nach Fig. 1 ein Modul zur Überprüfung der Vorratsbehälter (Ampullen) vorgesehen.

Mit diesem Modul wird die Positionierung der Ampulle auf richtigen Sitz in dem Gerät kontrolliert. Dies kann mit dem Auslesen des Speichers (in der Ampulle) kombiniert werden. Weiterhin muß die Erkennung eines Ampullenwechsels mit Zeit- und Datumsstempel dokumentiert werden.

Der mit der Ampulle verbundene Speicherchip wird beim Füllen der Ampulle mit Daten (Inhalt, Verfallsdatum, max. Abgabemenge, o.ä.) beschrieben.

Somit kann der Inhalt der Ampulle mit dem tatsächlich zu verabreichenden Impfstoff verglichen werden. Eine Überschreitung des Verfallsdatums ist erkennbar.

Für die Eingabe der Injektionsdaten gibt es entsprechend der Darstellung in der Fig. 1 eine definierte Schnittstelle, an die unterschiedlichste Eingabemedien angeschlossen werden können.

Die Schnittstelle wird so ausgelegt, daß die Eingabe über Tastatur, durch Auslesen programmierbarer Speicherchipkarten (z.B. KK-Karte) oder durch Empfang der Daten über eine Infrarot- bzw. Funkverbindung möglich ist. Die Eingabe ist ergonomisch auf die Erfordernisse abgestimmt.

Injektionsdaten sind z.B. Injektionsmenge, -intervalle und die zu erwartenden Aktivitäten des Patienten. Letzter Punkt dient vor allem der späteren Auswertung durch den behandelnden Arzt. Damit ist ihm die einmalige Möglichkeit gegeben, einen längeren Zeitverlauf zu überschauen und frühzeitig auf Veränderungen einzuwirken.

Die Datenausgabe erfolgt über ein LC - Display. Die Betriebszustände werden mit entsprechenden Piktogrammen angezeigt. Die Signalisierung spezieller Betriebszustände erfolgt mittels Piezo-Summer.

Die über einen bestimmten Zeitraum gesammelten Injektionsdaten können auf nichtflüchtige Speicher, z.B. auf Krankenkassenkarten, Drucker oder PC's ausgegeben werden, wo sie der statistischen Erfassung und Auswertung zugeführt werden.

Die Erfindung ist nicht auf das hier beschriebene Ausführungsbeispiel beschränkt. Vielmehr ist es möglich, durch Kombination und Modifikation der beschriebenen Merkmale weitere Ausführungsvarianten im Rahmen der nachstehenden Ansprüche zu realisieren.

### Bezugszeichenliste

- 1: Ansaugpumpe
- 2: Drucksensor
- 3: optischer Entfernungsmesser
- 4: Luftansaugringleitungssystem
- 5: Abschußdüse
- 6: Sensor zur Hautwiderstandsmessung
- 7: Epidermis
- 8: Dermis
- 9: Subcutis
- 10: Gewebeflüssigkeit
- 11: Muskelgewebe
- 12: Saugglocke
- 13: Haut
- 14: Kante
- 15: Ansaugglockenboden

## Patentansprüche

1. Injektionsgerät zum schmerzlosen Injizieren von Medikamenten in flüssiger Form, insbesondere von Insulin, mit einem Druckstrahlinjektor und einer Saugglocke (12) sowie elektronischer Rechentechnik, wobei eine Dosiereinrichtung mit Rückkopplung zu einer Rechnereinheit (MCU) zur Verwaltung der zur Anwendung vorgesehenen Medikamente, eine Ansaugeinrichtung (1) mit Rückkopplung zur Rechnereinheit zur Vorbereitung des Injektionsbereiches und eine Abschußeinrichtung (5) mit Rückkopplung zur Rechnereinheit zur zeit- und kraftgenauen Injektion verbunden sind, wobei die Rechnereinheit (MCU) mit Einrichtungen zur Dateneingabe und Datenausgabe verbunden ist,
**dadurch gekennzeichnet,**
**daß** Einrichtungen (2, 3, 6) zur Messung des elektrischen Hautwiderstandes und zur Ermittlung der Hautelastizität vorgesehen sind, die mit der Rechnereinheit wie Mikroprozessor (MCU) zur Auswertung der Meßergebnisse und zur Bestimmung und Variierung des Injektionsdruckes durch die Rechnereinheit verbunden sind.

2. Injektionsgerät nach Anspruch 1,
**dadurch gekennzeichnet,**
**daß** die Dateneingabe ein Tastaturfeld, einen Datenkanal zum Datenempfang von Meßgeräten wie Blutzuckermeßgeräten zur automatischen Dosierung, einen Datenkanal zur Eingabe von Injektionsdaten über eine Chipkarte über z.B. Medikamentenmenge und Intervalle der Injektionen sowie eine Zeitmeßeinrichtung aufweist.

3. Injektionsgerät nach Anspruch 2,
**dadurch gekennzeichnet,**
**daß** die Datenausgabe aus einem Display, einer akustischen Signaleinrichtung, einer Ausgabeeinrichtung wie Chipkarte, PC zur Ausgabe der durchgeführten Injektionen gebildet ist.

4. Injektionsgerät nach Anspruch 1,
**dadurch gekennzeichnet,**
**daß** kodierte, unverwechselbare Vorratsbehälter für Medikamente und sonstige Flüssigkeiten wie Verdünner, Reinigungsmittel vorgesehen sind, die mit der Rechnereinheit über Module verbunden sind, um deren kodierte Inhalte abrufbar zu machen.

## Claims

1. Injection device for the painless injection of liquid medicine , especially insuline, by a pressure ray injetor in combination with a vacuum extractor and electronic calculation technique, in which a measuring out-device with feedback to a calculation unit (MCU) administering the medicines to be given, a drawing in-device (1) with feedback to the calculation unit for preparing the injection area and a launching device (5) with feedback to the calculation unit are linked in order to ensure timely and otherwise correct injections, in which the calculation unit is linked to devices for in- an output of related data.
**Characterized by** the facts that
units (2,3,6) for measuring the electric resistor and elasticity of skin , which are linked with the calculation unit for the analysis of measuring results and for determination and variation of injection pressure by said calculation unit.

2. Injection device according to claim (1)
**Characterized by** the facts that,
the input of data disposes of a keyboard, a data channel for transmitting and receiving data from measuring devices such as devices for measuring blood sugar levels for automatic measuring out, a data channel for the input of injection data pertaining to e.g. doses of medicine and injection intervals via chip card as well as a time measuring device.

3. Injection device according to claim (2)
**Characterized by** the facts that,
the data output consists of a display, an acoustic set of signals, an output device such as chip card, computer for printing out the completed injections.

4. Injection device according to claim (1)
**Characterized by** the facts that,
Encoded distinctive containers for medicine and other liquids such as thinner, cleansing agents are in place, which are linked to the calculation unit via modules in order to make the encoded contents of said containers retrievable.

## Revendications

1. Appareil d'injection pour l'injection indolore des médicaments liquides, particulièrement d'insuline, à travers d'un injecteur de pression et une pompe aspirante (12) et avec de la technique à calculer électronique, qui sont liés à un dispositif de dosage avec couplage à un dispositif à calculer (MCU) pour administrer des médicaments qui doivent être appliqués ; un dispositif aspirant avec couplage au dispositif à calculer pour la préparation de cette partie de la peau dans laquelle les médicaments doivent être injectés ainsi qu'un dispositif pour le lancement (5) avec couplage au dispositif à calculer qui garantissent l'injection à temps et avec la force correcte. Le dispositif à calculer (MCU) est lié aux dispositifs pour l'entrée et la sortie des données.
**Caractérisés par** ce qui suit:
Il y a des dispositifs (2,3,6) qui mésurent la résistance et l'élasticité de la peau qui sont liés à le dispositif à calculer afin que les résultats du mésurage puissent être analysés et afin que la pression de l'injection par le dispositif à calculer puisse être déterminée et variée.

2. Appareil d'injection selon droit (1),
**caractérisé par** ce qui suit:
l'entrée de données dispose d'un clavier, d'un canal pour la transmission des données pour la réception des données en provenance des appareils de mésure comme des appareils pour le mésurage de la glycémie pour le dosage automatique, d'un canal de données pour l'entrée des données d'injection concernant, par example la quantité des médocaments et les intervals des injections à travers une chip-card (carte microprocesseur), ainsi que d'un appareil pour le mésurage du temps.

3. Appareil d'injection selon droit (2)
**caractérisé par** ce qui suit:
La sortie des données se compose d'un display, une installation de signalisation acoustique comme chip-card, ordinateur pour la sortie des injections effectuées.

4. Appareil d'injection selon droit (1)
**caractérisé par** ce qui suit:
Des conteneurs de réserve codés et qui ne peuvent pas être confondus qui sont prévus pour des médicaments et pour autres liquides comme des diluants et des détergents, qui sont liés au dispositif à calculer à travers des moduls afin que les contenues codés puissent être rappelés.
